(19) Europäisches Patentamt
European Patent Office
Office européen des brevets

(11) Veröffentlichungsnummer: **0 252 487 B1**

(12) **EUROPÄISCHE PATENTSCHRIFT**

(45) Veröffentlichungstag der Patentschrift: **08.05.91**

(51) Int. Cl.5: **A61J 3/00**, A61J 1/03, A61K 9/20

(21) Anmeldenummer: **87109766.3**

(22) Anmeldetag: **07.07.87**

(54) **Konfektionspackungen, enthaltend Arzneimittelkombinationen für zeitlich abgestufte Anwendung.**

(30) Priorität: **11.07.86 DE 3623331**

(43) Veröffentlichungstag der Anmeldung: **13.01.88 Patentblatt 88/02**

(45) Bekanntmachung des Hinweises auf die Patenterteilung: **08.05.91 Patentblatt 91/19**

(84) Benannte Vertragsstaaten: **AT BE CH DE ES FR GB GR IT LI LU NL SE**

(56) Entgegenhaltungen:
**DE-A- 2 834 226**
**US-A- 2 317 860**
**US-A- 3 207 299**
**US-A- 4 553 670**

(73) Patentinhaber: **HOECHST AKTIENGESELLSCHAFT**
**Postfach 80 03 20**
**W-6230 Frankfurt am Main 80(DE)**

(72) Erfinder: **Weithmann, Klaus Ulrich, Dr.**
**Am Domherrnwald 18**
**W-6238 Hofheim am Taunus(DE)**
Erfinder: **Seiffge, Dirk, Dr.**
**Hauptstrasse 2**
**W-6309 Münzenberg(DE)**

**EP 0 252 487 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In vielen Fällen wird den Patienten von ärztlicher Seite die Einnahme von mehreren Arzneimitteln verordnet, und zwar in der Weise, daß die Arzneimittel zu gleicher Zeit oder mit zeitlichem Abstand genommen werden sollen. Für den Gebrauch in Krankenhäusern sind hierfür eigene Schachteln entwickelt worden, in denen die Summe der Arzneimittel für einen Tag den Patienten auf einmal übergeben wird. Hierbei sind Fehlermöglichkeiten in der Einnahme nicht ausgeschlossen, da es immer vorkommen kann, daß der Patient die Einnahme der einen oder anderen Tablette vergißt oder den verordneten Zeitabstand nicht einhält.

Es hat sich auch herausgestellt, daß in bestimmten Fällen eine mit zeitlichem Abstand aufeinanderfolgende Anwendung von zwei pharmakologischen Wirkstoffen zu überraschenden und herausragenden Effekten führt, wobei also eine Komponente zuerst freigesetzt wird. So bewirkt eine mit zeitlicher Abstufung aufeinanderfolgende Anwendung von A) Xanthinderivaten bzw. deren wirksamen Metaboliten einerseits und B) Acetylsalicylsäure bzw. deren pharmakologisch verträglichen Salzen andererseits in einer bestimmten Reihenfolge eine überaus starke Verbesserung der Therapie von Krankheiten, die durch gestörte Blutbestandteile, insbesondere Thrombozyten bzw. Erythrozyten, aber auch Leukozyten verursacht bzw. gekennzeichnet sind. Die zeitlich abgestufte Anwendung der Xanthinderivate, insbesondere Pentoxifyllin, die erst nach l0 Minuten bis 4 Stunden von der Anwendung der Acetylsalicylsäure bzw. ihres Salzes gefolgt wird, führt zu viel stärkeren Effekten als wenn die Kombination aus den beiden Einzelsubstanzen gleichzeitig verabfolgt wird, wo sogar eine Verminderung dieser Wirkung eintritt.

Es sind schon Konfektionspackungen beschrieben worden, die verschiedene Kammern enthalten und außerdem eine daneben befindliche Einnahmeanweisung (US-A- 4553670). Durch diese Konfektionspackungen lassen sich Irrtümer des Patienten bezüglich der richtigen zeitlichen Einnahme zwar vermindern, aber nicht ausschließen. Die US-A- 2 317 860 beschreibt Konfektionspackungen mit zwei gleichgestalteten Dosierungseinheiten, die in einer Kammer enthalten sind. Durch solche Konfektionspackungen wird auch nicht vermieden, daß die beiden Dosierungseinheiten bei Bewegungen der Packung, wie sie unvermeidlich sind, durch Abrieb beschädigt werden.

Die Erfindung hat sich nun zum Ziel gesetzt, die Sicherheit für den Patienten bei der notwendigen Verabreichung von mehreren Tabletten, wie sie schon vom Hersteller von Arzneimitteln vorausgesehen werden kann, gegen Verwechslungen noch weiter zu erhöhen.

Gegenstand der vorliegenden Erfindung sind nun Konfektionspackungen, die Kammern mit mindestens zwei festen, nicht mechanisch verbundenen Dosierungseinheiten von verschiedenen Arzneimitteln enthalten, die sich vorzugsweise erkennbar, z.B. in ihrer Größe, Gewicht, Form und/oder Färbung voneinander unterscheiden. Wo eine in zeitlichem Abstand erfolgende Freigabe im Körper notwendig ist, kann das eine Arzneimittel in verzögerter Form vorliegen. Eine bevorzugte Ausführungsform besteht darin, daß die verschiedenen Dosierungseinheiten durch räumliche Gestaltung der Kammern, vorzugsweise eine stegartige Aufwölbung (l) (s. Figur 8), auch mechanisch voneinander getrennt und so fest verankert sind, daß die räumliche Trennung gewährleistet bleibt und keine Beschädigung durch Abrieb erfolgt.

Der Begriff "verschiedene Arzneimittel" bezeichnet zwar vor allem solche, die verschiedene Wirkstoffe enthalten, jedoch soll er auch Kombinationen umfassen, die zwar denselben Wirkstoff enthalten, aber in verschiedenen Darbietungsformen, z.B. in nicht-retardierter und retardierter Form getrennt vorliegen.

Eine bevorzugte Ausführungsform sieht Konfektionspackungen vor, die feste Dosierungseinheiten von je A) einem Xanthinderivat der Formel I bzw. II (s. Anspruch 8) bzw. Pro-Drugs von Oxoalkyl- bzw. Hydroxyalkylxanthinen, bzw. deren wirksamen Metaboliten einerseits und B) O-Acetylsalicylsäure bzw. deren pharmakologisch verträglichen Salzen nebeneinander enthalten, wobei die Komponente B in retardierter Form vorliegt und jede der beiden Komponenten A) und B) vorzugsweise mit einem pharmazeutischen Träger verarbeitet ist. In Formel I ist einer der Reste $R^1$ und $R^3$ eine geradkettige Alkyl-, ($\omega$-l)-Oxoalkyl- oder ($\omega$-l)-Hydroxyalkylgruppe mit 3 bis 8 C-Atomen und die beiden anderen Reste $R^2$ und $R^3$ oder $R^1$ und $R^2$ stellen geradkettige oder verzweigte Alkylgruppen mit l bis 8 C-Atomen in der Position von $R^1$ und $R^3$ und l bis 4 C-Atomen in der Position von $R^2$ dar, wobei die Summe der C-Atome dieser beiden Alkylsubstituenten höchstens l0 beträgt und in Formel II R ein Alkylrest mit l bis 4 C-Atomen ist. Bevorzugt sind dabei solche Xanthinverbindungen der Formel I zugegen, in denen $R^1$ oder $R^3$ einen Alkyl-, ($\omega$-l)-Oxoalkyl- oder ($\omega$-l)-Hydroxyalkylrest mit 5 oder 6 C-Atomen bedeutet und die beiden Alkylsubstituenten $R^2$ und $R^3$ bzw. $R^1$ und $R^2$ zusammen 2 bis 6 C-Atome umfassen. Von diesen sind wiederum solche bevorzugt, in denen sich in der Position von $R^1$ oder $R^3$ eine Hexyl-, 5-Oxohexyl- oder 5-Hydroxyhexylgruppe befindet und insbesondere das l-Hexyl-3,7-dimethyl-xanthin, l-(5-Hydroxyhexyl)-3,7-dimethylxanthin, l-(5-Oxohexyl)-3,7-dimethyl-xanthin, l,3-Dimethyl-7-(5-hydroxyhexyl)-xanthin, l,3-Dimethyl-7-(5-oxohexyl)-xanthin, l-(5-Hydroxyhexyl)-3-methyl-7-propylxanthin oder l-(5-Oxohexyl)-3-methyl-7-propylxanthin.

Das Xanthinderivat kann auch in Prodrug-Form als acetalisiertes Oxoalkylxanthin vorhanden sein, in dem wenigstens eine Carbonylgruppe durch das Strukturelement der Formel

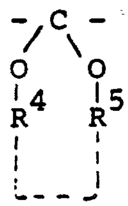

(III)

ersetzt ist oder als O-acyliertes Hydroxyalkylxanthin mit dem Strukturelement der Formel $R^6$-CO-O- (IV), wobei $R^4$ und $R^5$ jeweils eine Alkylgruppe mit bis zu 4 C-Atomen oder zusammen eine Äthylen-, Trimethylen- oder Tetramethylengruppe darstellen und $R^6$ einen Alkylrest mit bis zu 4 C-Atomen, Phenyl, substituiertes Phenyl, Pyridyl oder substituiertes Pyridyl bedeutet.

Solche Konfektionspackungen eignen sich ganz besonders für Mittel, die aufgrund ihrer überadditiven Effekte zur antithrombotischen, blutflußfördernden, entzündungshemmenden, schmerzstillenden, antiaggregatorischen und cytostatischen Therapie bzw. Prophylaxe bestimmt sind. Wegen des überadditiven Effektes bei der zeitlich verzögerten Freisetzung können die anzuwendenden Mengen an z.B. Xanthinderivat und Acetylsalicylsäure auf solche Mengen reduziert werden, die bei ihrer alleinigen Verabreichung eine nur minimale pharmakologische Wirkung zeigen, so daß gleichzeitig Nebenwirkungen, die von hohen Dosen dieser Wirkstoffe hervorgerufen werden, verringert werden. Dies ist deswegen von großer Bedeutung, weil bekanntlich Acetylsalicylsäure in den üblichen Dosierungen unerwünschte Nebenwirkungen wie Asthma, allergische Urtikaria, analgetische Nephropathie sowie Magen- und Darmulcera hervorrufen kann. Die Erfindung ermöglicht es nun, auch diese beiden Wirkstoffe in der erforderlichen Weise mit Sicherheit zu dosieren, also Fehldosierungen auszuschließen.

Analog wie Kombinationen von Xanthinderivaten und Acetylsalicylsäure lassen sich auch solche von A) Pyrimidopyrimidinen der Formel IV (s. Anspruch 9), wie Dipyridamol - vgl. DE-OS 35 I5 874 - mit B) O-Acetylsalicylsäure bzw. deren pharmazeutisch verträglichen Salzen erfindungsgemäß für die gleiche Indikation darbieten. In Formel IV stellt wenigstens einer der Reste $R^6$ und $R^8$ den Rest -N($CH_2$-$CHR^{10}$-OH$)_2$ mit $R^{10}$ = Wasserstoff oder Methyl und wenigstens einer der Reste $R^7$ und $R^9$ den Rest

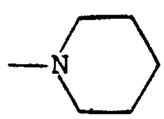

dar, der in p-Stellung zum Stickstoffatom auch durch Sauerstoff unterbrochen sein kann. Statt dessen kann auch ein wirksamer Metabolit und/oder ein wirksames Salz zugegen sein. Dabei soll das Gewichtsverhältnis der Komponente A) zur Komponente B) größer als 0,5 sein. Diese beiden Komponenten können mit oder ohne C) einen pharmazeutischen Träger verarbeitet sein. Derartige Kombinationen dienen ebenfalls zur zeitlich abgestuften Anwendung in der Therapie von Krankheiten, die durch gestörte Blutfunktionen bzw. Blutbestandteile, insbesondere Thrombocyten bzw. Erythrocyten verursacht bzw. gekennzeichnet sind, wobei beabsichtigt ist, daß die Komponente A) zuerst freigesetzt wird.

Die Erfindung ermöglicht es also, die Therapie mit Mehrstoffkombinationen erfolgreich zu gestalten, indem die Einnahme der Komponenten dieser Arzneimittelkombination durch den Patienten zuverlässig. d.h. gleichzeitig, erfolgt, wobei, z.B. durch Einarbeitung von Retardmitteln in ein Arzneimittel, auch ein zeitlicher Zusammenhang zwischen den Wirkungen der verschiedenen Arzneimittel hergestellt werden kann.

Einige z.T. bisher unübliche Gestaltungsformen der erfindungsgemäßen Kombinationen sind in den Figuren I bis 9 in Ansicht wiedergegeben. Bevorzugt sind die einzelnen Dosierungseinheiten so gestaltet, daß sie für sich oder in der Kombination miteinander eine geometrisch (stereometrisch) symmetrische Gestalt, z.B die übliche Tablettenform oder Kugel-, Zylinder-, Scheiben-, Stab-, Ellipsoid-, Kegel-, Doppelkegel- oder Doppelkegelstumpfform haben oder eine Kombination von Scheibe und Ring sind. So kann die Sicherheit gegen Verwechslungen bei der Einnahme nach einer weiteren bevorzugten Ausführungsform dadurch erhöht werden, daß die einzelnen Dosierungseinheiten als Teile von geometrischen (stereometrischen) Formen dargeboten werden, z.B. als zwei gleichgroße, aber spiegelbildlich angeordnete

und z.B. unterschiedlich gefärbte Halbkugeln bzw., bei einem Multikomponentensystem, als Kugelsegmente. Besonders vorteilhaft ist es, wenn die Teile so beschaffen sind, daß sie zusammengesteckt werden können (Schlüssel-Schloß-Prinzip siehe Figuren 5 und 7).

Eine gute Sicherheit gegen Verwechslungen bei der Einnahme kann auch erreicht werden, wenn die Dosierungseinheiten, z.B. in einer Blisterpackung, derart angeordnet werden, daß sie gleichzeitig entnommen werden können, z.B. gemeinsam in einer Kammer vorliegen (s. Figur 8), evtl. auch in Form von Zylindern oder Halbkugeln oder als Kombination dargeboten werden (s. z.B. Figuren I bis 7). Zur räumlichen Trennung der Dosierungseinheiten können auch zusätzliche Folien (3) zu den zur Herstellung der Blisterpackungen verwendeten Folien (2) und (4) herangezogen werden (s. Figuren 8 und 9).

## Ansprüche

1. Konfektionspackungen die kammern aufweisen, in denen mindestens zwei feste, nicht mechanisch verbundene Dosier-einheiten vorhanden sind dadurch gekennzeichnet, daß die Dosierungseinheiten aus verschiedenen Arzneimitteln bestehen und daß sie sich erkennbar unterscheid.

2. Konfektionspackungen nach Anspruch 1, dadurch gekennzeichnet, daß sich die Dosierungseinheiten in ihrer Größe, Form und/oder Färbung voneinander unterscheiden.

3. Konfektionspackungen nach einem oder mehreren der Ansprüche 1 bis 2, dadurch gekennzeichnet, daß die verschiedenen Dosierungsformen durch räumliche Gestaltung, vorzugsweise eine stegartige Aufwölbung (1), auch mechanisch voneinander getrennt sind.

4. Konfektionspackungen nach einem oder mehreren der Ansprüche 1 bis 3, dadurch gekennzeichnet, daß die einzelnen Dosierungseinheiten so gestaltet sind, daß sie für sich oder in der Kombination miteinander eine geometrisch symmetrische Gestalt haben oder eine Kombination von Scheibe und Ring sind.

5. Konfektionspackungen nach einem oder mehreren der Ansprüche 1 bis 4, dadurch gekennzeichnet, daß die Dosierungseinheiten der Arzneimittel Teile von stereometrischen Formen sind.

6. Konfektionspackungen nach einem oder mehreren der Ansprüche 1 bis 5, dadurch gekennzeichnet, daß die Dosierungseinheiten in einer Blisterpackung vorliegen, worin sie vorzugsweise durch zusätzliche Folien (3) voneinander getrennt sind.

7. Konfektionspackungen nach einem oder mehreren der Ansprüche 1 bis 6, dadurch gekennzeichnet, daß sie feste Dosierungseinheiten von je A) einem Xanthinderivat der Formel (I) bzw. (II)

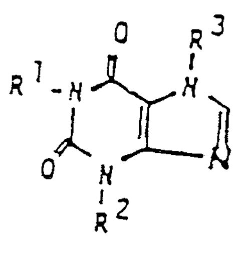 (I) 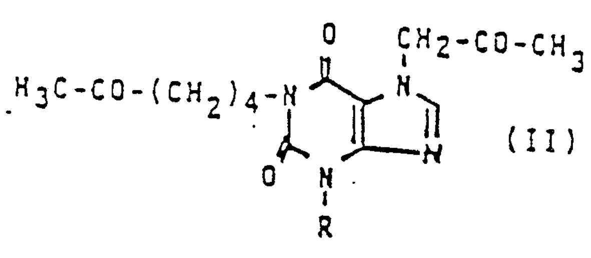 (II)

bzw. Pro-Drugs von Oxoalkyl- bzw. Hydroxylalkylxanthinen, bzw. deren wirksamen Metaboliten einerseits und B) O-Acetylsalicylsäure bzw. deren pharmakologisch verträglichen Salzen nebeneinander enthalten, wobei in Formel I einer der Reste $R^1$ und $R^3$ eine geradkettige Alkyl-, (ω-l)-Oxoalkyl-oder (ω-l)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit I bis 8 C-Atomen in der Position von $R^1$ und $R^3$ und bis 4 C-Atomen in der Position von $R^2$ darstellen, wobei die Summe der C-Atome dieser beiden Alkylsubstituenten höchstens I0 beträgt, und in Formel II R ein Alkylrest mit I bis 4 C-Atomen ist, und die Komponente B in retardierter Form vorliegt und jede der beiden Komponenten A) und B) vorzugsweise mit einem pharmazeutischen Träger verarbeitet ist.

8. Konfektionspackungen nach einem oder mehreren der Ansprüche 1 bis 6, dadurch gekennzeichnet, daß sie feste Dosierungseinheiten enhalten von je A) einem Pyrimido-pyrimidin der Formel IV

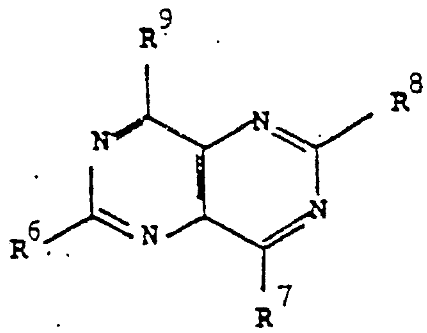

worin wenigstens einer der Reste $R^6$ und $R^3$ den Rest $-N(CH_2-CHR^{10}-OH)_2$ mit $R^{10}$ = Wasserstoff oder Methyl und wenigstens einer der Reste $R^7$ und $R^9$ den Rest

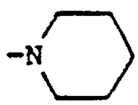

darstellt, der in p-Stellung zum Stickstoffatom auch durch Sauerstoff unterbrochen sein kann,
bzw. dessen wirksame Metabolite und/oder Salze und
B) andererseits O-Acetylsalicylsäure bzw. deren pharmazeutisch verträgliche Salze,
wobei das Gewichtsverhältnis der Komponente A) zur Komponente B) größer als 0,5 ist, mit oder ohne C) einen pharmazeutischen Träger.

## Claims

1. A dispensing pack which has chambers in which there are at least two solid, not mechanically connected dosage units, which dosage units are composed of diverse pharmaceuticals and are recognizably different.

2. A dispensing pack as claimed in claim 1, wherein the dosage units differ from one another in their size, shape and/or color.

3. A dispensing pack as claimed in one or more of claims 1 or 2, wherein the various dosage forms are also mechanically separated from one another by the spatial configuration, preferably a ridge-like arching (1).

4. A dispensing pack as claimed in one or more of claims 1 to 3, wherein the shapes of the individual dosage units are such that they have a geometrically symmetrical shape, either alone or in combination with one another, or are a combination of disk and ring.

5. A dispensing pack as claimed in one or more of claims 1 to 4, wherein the dosage units of the pharmaceuticals are parts of stereometric shapes.

6. A dispensing pack as claimed in one or more of claims 1 to 5, wherein the dosage units are located in a blister pack in which they are preferably separated from one another by additional sheets (3).

7. A dispensing pack as claimed in one or more of claims 1 to 6, which contains, side by side, solid dosage units of, in each case, A) a xanthine derivative of the formula (I) or (II)

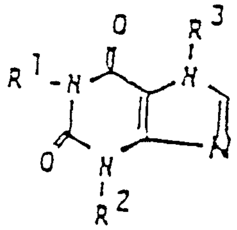
(I)

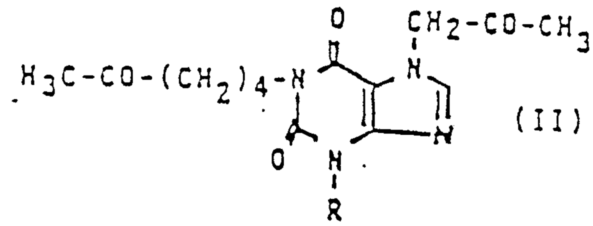
(II)

or prodrugs of oxoalkyl- or hydroxyalkylxanthines, or their active metabolites on the one hand, and B) O-acetylsalicylic acid or its pharmacologically tolerated salts, where in formula I one of the radicals $R^1$ and $R^3$ represents a straight-chain alkyl, ($\omega$-1)-oxoalkyl or ($\omega$-1)-hydroxyalkyl group having 3 to 8 carbon atoms, and the two other radicals $R^2$ and $R^3$, or $R^1$ and $R^2$, represent straight-chain or branched alkyl groups having 1 to 8 carbon atoms in the position of $R^1$ and $R^3$, and 1 to 4 carbon atoms in the position of $R^2$, the total of the carbon atoms in these two alkyl substituents not exceeding 10, and in formula II R being an alkyl radical having 1 to 4 carbon atoms, and the component B being present in delayed release form, and each of the two components A) and B) preferably being processed with a pharmaceutical vehicle.

8. A dispensing pack as claimed in one or more of claims 1 to 6, which contains solid dosage units of, in each case, A) a pyrimidopyrimidine of the formula IV

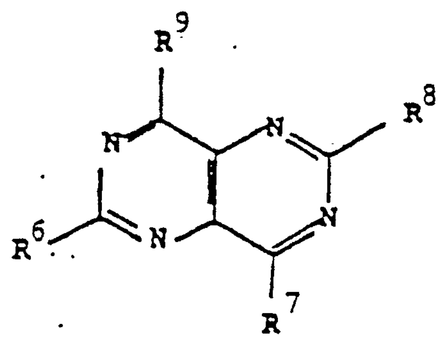

in which at least one of the radicals $R^6$ and $R^8$ represents the radical $-N(CH_2-CHR^{10}-OH)_2$ with $R^{10}$ = hydrogen or methyl, and at least one of the radicals $R^7$ and $R^9$ represents the radical

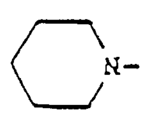

which can also be interrupted by oxygen in the p-position to the nitrogen atom, or its active metabolites and/or salts, and B) on the other hand, O-acetylsalicylic acid or its pharmaceutically tolerated salts, the ratio by weight of component A) to component B) being greater than 0.5, with or without C) a pharmaceutical vehicle.

**Revendications**

1. Conditionnements présentant des compartiments dans lesquels sont présents au moins deux doses unitaires solides, non associées mécaniquement, caractérisés en ce que les doses unitaires consistent en des médicaments différents et en ce qu'elles se distinguent de façon perceptible.

2. Conditionnements selon la revendication 1, caractérisés en ce que les doses unitaires se distinguent l'une de l'autre (les unes des autres) par leur taille, forme et/ou coloration.

3. Conditionnements selon la revendication 1 ou 2, caractérisés en ce que les diverses doses unitaires

sont également séparées mécaniquement l'une de l'autre (les unes des autres) par une configuration spatiale, de préférence une bombement (1) de type nervure.

**4.** Conditionnements selon une ou plusieurs des revendications 1 à 3, caractérisés en ce que les doses unitaires individuelles sont constituées de manière à avoir, en soi ou dans l'association l'une avec l'autre (les unes avec les autres) une symétrie géométrique, ou à être une association de disque et d'anneau.

**5.** Conditionnements selon une ou plusieurs des revendications 1 à 4, caractérisés en ce que les doses unitaires des médicaments sont des éléments de formes stéréométriques.

**6.** Conditionnements selon une ou plusieurs des revendications 1 à 5, caractérisés en ce que les doses unitaires sont présentes dans un blister dans lequel elles sont de préférence séparées l'une de l'autre (les unes des autres) par des pellicules supplémentaires (3).

**7.** Conditionnements selon une ou plusieurs des revendications 1 à 6, caractérisés en ce qu'ils contiennent l'une à côté de l'autre des doses unitaires solides, respectivement, d'une part A) d'un dérivé de xanthine de formule (I) ou (II)

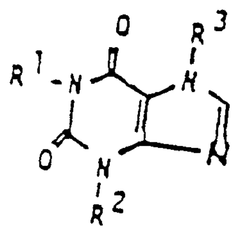
(I)

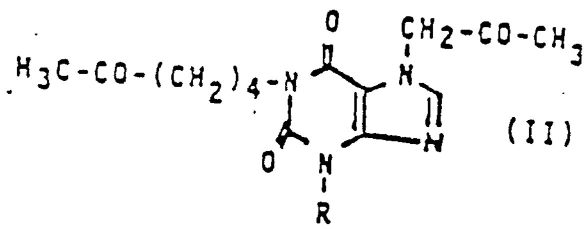
(II)

ou de précurseurs de médicaments d'oxoalkyl- ou hydroxyalkyl-xanthines ou de leurs métabolites actifs, et d'autre part B) d'acide O-acétylsalicylique ou de ses sels pharmacologiquement acceptables, dans la formule I l'un des radicaux $R^1$ et $R^3$ représentant un groupe alkyle, ($\omega$-1)-oxoalkyle ou ($\omega$-1)-hydroxyalkyle à chaîne droite à 3-8 atomes de carbone, et les deux autres radicaux $R^2$ et $R^3$ ou $R^1$ et $R^2$ représentent des groupes alkyle à chaînes droites ou ramifiées ayant de 1 à 8 atomes de carbone en la position de $R^1$ et $R^3$ et de 1 à 4 atomes de carbone en la position de $R^2$, la somme des atomes de carbone de ces deux substituants alkyle étant de 10 au maximum, et dans la formule II, R étant un radical alkyle ayant de 1 à 4 atomes de carbone, et le composant B se trouvant sous forme retard et chacun des deux composants A) et B) étant de préférence travaillé avec un véhicule pharmaceutique.

**8.** Conditionnements selon une ou plusieurs des revendications 1 à 6, caractérisés en ce qu'ils contiennent des doses unitaires solides, respectivement, de A) une pyrimido-pyrimidine de formule IV

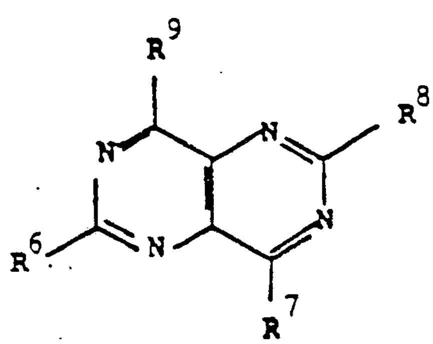

dans laquelle au moins l'un des radicaux $R^6$ et $R^8$ représente le radical $-N(CH_2-CHR^{10}-OH)_2$, $R^{10}$ étant un atome d'hydrogène ou le groupe méthyle, et au moins l'un des radicaux $R^7$ et $R^9$ représente le radical

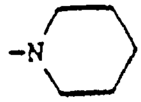

qui peut également être interrompu par un atome d'oxygène en position para par rapport à l'atome d'azote,
ou des métabolites et/ou sels actifs de celle-ci, et
B) d'autre part d'acide O-acétylsalicylique ou de ses sels pharmaceutiquement acceptables,
le rapport pondéral du composant A) au composant B) étant supérieur à 0,5, avec ou sans
C) un véhicule pharmaceutique

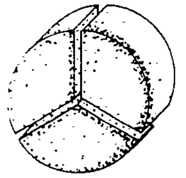
FIG.1

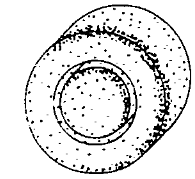
FIG.2

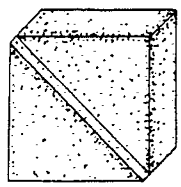
FIG.3

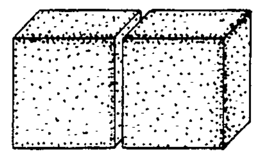
FIG.4

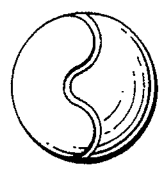
FIG.5

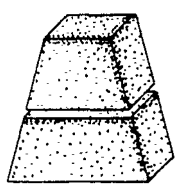
FIG.6

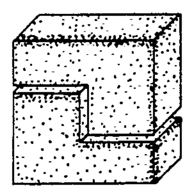
FIG.7

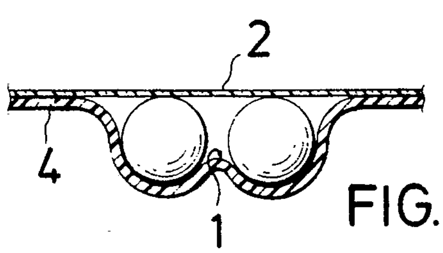

FIG.8

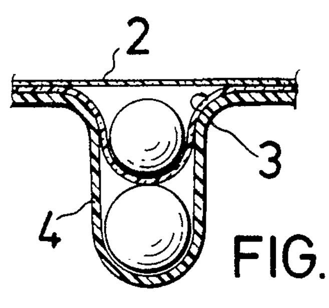

FIG.9